(19) Europäisches Patentamt
European Patent Office
Office européen des brevets

(11) **EP 4 383 263 A1**

(12) **EUROPEAN PATENT APPLICATION**
published in accordance with Art. 153(4) EPC

(43) Date of publication:
**12.06.2024 Bulletin 2024/24**

(21) Application number: **22898484.5**

(22) Date of filing: **16.11.2022**

(51) International Patent Classification (IPC):
**G16C 20/30** $^{(2019.01)}$    **G01N 3/00** $^{(2006.01)}$

(52) Cooperative Patent Classification (CPC):
**G01N 3/00; G16C 20/30;** Y02T 90/00

(86) International application number:
**PCT/JP2022/042597**

(87) International publication number:
**WO 2023/095700 (01.06.2023 Gazette 2023/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.11.2021   JP 2021191460**

(71) Applicant: **Sumitomo Riko Company Limited**
**Komaki-shi, Aichi 485-8550 (JP)**

(72) Inventors:
• **WATANABE Naoki**
  **Komaki-shi, Aichi 485-8550 (JP)**
• **KASAI Seiji**
  **Komaki-shi, Aichi 485-8550 (JP)**
• **YAJIMA Takashi**
  **Komaki-shi, Aichi 485-8550 (JP)**
• **KOYAMA Toshiyuki**
  **Nagoya-shi, Aichi 464-8601 (JP)**

(74) Representative: **Kramer Barske Schmidtchen**
**Patentanwälte PartG mbB**
**European Patent Attorneys**
**Landsberger Strasse 300**
**80687 München (DE)**

(54) **DEVICE FOR SIMULATING VISCOELASTIC PROPERTY OF POLYMER COMPOSITE MATERIAL**

(57)     A device (1) for simulating a viscoelastic property of a polymer composite material in which a filler is dispersed in a matrix polymer includes: a storage unit (3) that stores a simulation model (M) formed by a model of a matrix phase (B) of the matrix polymer, a model of the filler (A), and a model of an interface phase (C) of the matrix polymer; and an analysis unit (4) that analyzes a storage elastic modulus and a loss elastic modulus of the polymer composite material by solving a dynamic equilibrium equation in a case where a vibration strain (U(t)) is applied to the simulation model (M). The model of the interface phase C is a model in which a storage elastic modulus and a loss elastic modulus in the interface phase C are defined using a phase field variable (x) for setting a ratio between a rubbery state and a glassy state of the matrix polymer.

FIG. 4

EP 4 383 263 A1

**Description**

TECHNICAL FIELD

The present invention relates to a device for simulating a viscoelastic property of a polymer composite material.

BACKGROUND ART

Patent Literature 1 describes simulation regarding a viscoelastic property of a polymer composite material. The polymer composite material to be simulated is a material in which a filler is dispersed in a matrix polymer. In this type of polymer composite material, the matrix polymer has different properties between a site present as a matrix phase and a site of an interface phase close to the filler. Then, the viscoelastic properties of the matrix phase and the interface phase are set as a finite element model.

PRIOR ART LITERATURE

Patent Literature

Patent Literature 1: JP 6637845 B2

SUMMARY OF INVENTION

PROBLEMS TO BE SOLVED BY INVENTION

In a case where the viscoelastic properties are set as a finite element model, the degree of freedom is low. As the polymer composite material, there are a polymer composite material in which the interface phase has an identical viscoelastic property over the entire region, a polymer composite material in which a viscoelastic property is different for each region, and the like. In addition, in the polymer composite material, the thickness of the interface phase is also important. When the viscoelastic property of each region in the interface phase can be varied depending on the thickness of the interface phase, the viscoelastic property of the polymer composite material can be obtained with higher accuracy.
The present invention has been made in view of such a background, and is directed to providing a device for simulating a viscoelastic property of a polymer composite material using a model different from the finite element model.

MEANS FOR SOLVING PROBLEMS

One aspect of the present invention is a device for simulating a viscoelastic property of a polymer composite material in which a filler is dispersed in a matrix polymer, the device comprising:

a storage unit that stores a simulation model formed by a model of a matrix phase of the matrix polymer, a model of the filler, and a model of an interface phase of the matrix polymer located between the matrix phase of the matrix polymer and the filler; and
an analysis unit that analyzes a storage elastic modulus and a loss elastic modulus of the polymer composite material by solving a dynamic equilibrium equation in a case where a vibration strain is applied to the simulation model, wherein the model of the interface phase is a model in which a storage elastic modulus and a loss elastic modulus in the interface phase are defined using a phase field variable for setting a ratio between a rubbery state and a glassy state of the matrix polymer.

EFFECTS OF INVENTION

According to the simulation device described above, the model of the interface phase is a model defined using the phase field variable. By varying the phase field variable, the property of the model of the interface phase can be freely set. Thus, the model of the interface phase has a high degree of freedom and is easily set. As a result, the viscoelastic property of the polymer composite material can be simulated with high accuracy.

BRIEF DESCRIPTION OF DRAWINGS

Fig. 1 is a view illustrating a cross-sectional image of a polymer composite material as a target of a simulation device in a first embodiment.

Fig. 2 is a diagram for explaining a dynamic viscoelastic property.

Fig. 3 is a diagram illustrating a configuration of the simulation device according to the first embodiment.

Fig. 4 is a flowchart showing processing by a simulation model creation processing unit constituting the simulation device.

Fig. 5 is a view illustrating a simulation model of a polymer composite material, and is a view illustrating a model in which positions of a filler, a matrix phase, and an interface phase as respective elements are defined.

Fig. 6 is an enlarged view of the simulation model illustrated in Fig. 5, and is a diagram illustrating distributions of the filler, the matrix phase, and the interface phase at each position (pixel).

Fig. 7 is a chart showing a model type of each element of the polymer composite material in the first embodiment.

Fig. 8 is a diagram illustrating a generalized Maxwell model as a model type of the matrix phase in the first embodiment.

Fig. 9 is a diagram showing a phase field variable of the interface phase in the first embodiment.

Fig. 10 is a schematic diagram showing elastic moduli of the filler, the matrix phase, and the interface phase in the first embodiment.

Fig. 11 is a diagram illustrating states before and after a vibration strain is applied to the simulation model in processing by an analysis unit constituting the simulation device.

Fig. 12 is a diagram showing viscoelastic properties of the polymer composite material as a result by the analysis unit in the first embodiment.

Fig. 13 is a diagram showing a phase field variable of an interface phase in a second embodiment.

Fig. 14 is a schematic diagram showing elastic moduli of a filler, a matrix phase, and an interface phase in the second embodiment.

Fig. 15 is a schematic diagram showing elastic moduli of a filler, a matrix phase, and an interface phase in a third embodiment.

Fig. 16 is a chart showing a model type of each element of a polymer composite material in a fourth embodiment.

Fig. 17 is a diagram showing phase field variables of a matrix phase and an interface phase in the fourth embodiment.

MODE FOR CARRYING OUT INVENTION

(First Embodiment)

1. Simulation target P

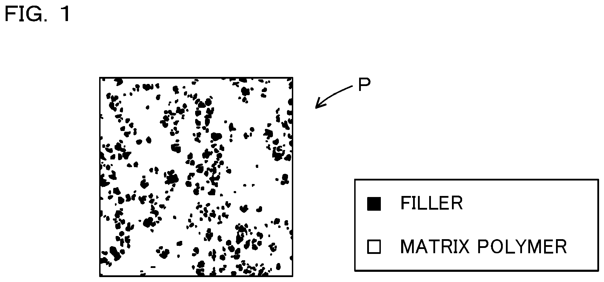

A simulation target P will be described with reference to Fig. 1. The simulation target P is a polymer composite material. The polymer composite material, such as a rubber composite material, is used in a variety of fields, for example, tires, dampers, shoes, sporting goods, or the like. The polymer composite material is formed by dispersing a filler depending on a purpose in a matrix polymer. For example, in a tire, a vulcanized rubber reinforced by dispersing a nanofiller as a filler such as carbon black in a matrix polymer is used.

In addition, an aggregated filler is non-uniformly distributed in the polymer composite material due to aggregation of the nanofiller. Thus, as illustrated in Fig. 1, the simulation target P is a polymer composite material in which a filler is non-uniformly distributed. However, the simulation target P may be a polymer composite material in which fillers are uniformly distributed.

Furthermore, a tire or the like repeatedly receives a deformation load. Thus, the simulation target P is a polymer composite material that repeatedly receives a vibration strain for causing deformation. That is, the simulation target P is a polymer composite material directly considering a non-uniform structural form, for which a dynamic viscoelastic property is to be evaluated.

In addition, the simulation target P has a meso scale, that is, a definition region having 10 nm to 100 $\mu$m as one side. A size of the definition region of the simulation target P is set such that an effect caused by the dispersion state of the filler on the viscoelastic property of the polymer composite material can be grasped.

2. Viscoelastic property

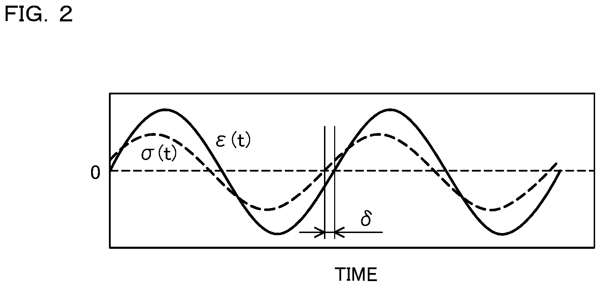

The viscoelastic property of the polymer composite material will be described with reference to Fig. 2. A strain $\varepsilon(t)$ oscillating at each angular frequency $\omega$ is applied to the polymer composite material, and a time-responsive stress $\sigma(t)$ is measured. The strain $\varepsilon(t)$ and the responsive stress $\sigma(t)$ in this case take on behaviors shown in Fig. 2. That is, the responsive stress $\sigma(t)$ is delayed in phase by $\delta$ with respect to the strain $\varepsilon(t)$. The strain $\varepsilon(t)$ is expressed by Equation (1), and the responsive stress $\sigma(t)$ is expressed by Equation (2).
[Math. 1]

$$\varepsilon(t) = \varepsilon^{(0)}sin(\omega t) \qquad \cdots \quad (1)$$

[Math. 2]

$$\sigma(t) = \sigma^{(0)}sin(\omega t + \delta)$$
$$= \sigma^{(0)}cos(\delta)sin(\omega t) + \sigma^{(0)}sin(\delta)cos(\omega t) \qquad \cdots \quad (2)$$

In this case, a storage elastic modulus $G'(\omega)$ is expressed by Equation (3). The storage elastic modulus $G'(\omega)$ is a value obtained by dividing the first term of the second expression of Equation (2) by an amplitude $\varepsilon^{(0)}$ of the strain $\varepsilon(t)$. The storage elastic modulus $G'(\omega)$ represents an elastic portion of the polymer composite material.
[Math. 3]

$$G'(\omega) = \frac{\sigma^{(0)}cos(\delta)}{\varepsilon^{(0)}} \qquad \cdots \quad (3)$$

A loss elastic modulus $G''(\omega)$ is expressed by Equation (4). That is, the loss elastic modulus $G''(\omega)$ is a value obtained by dividing the second term of the second expression of Equation (2) by the amplitude $\varepsilon^{(0)}$ of the strain $\varepsilon(t)$. The loss elastic modulus $G''(\omega)$ represents a viscous portion of the polymer composite material.
[Math. 4]

$$G''(\omega) = \frac{\sigma^{(0)}sin(\delta)}{\varepsilon^{(0)}} \qquad \cdots \quad (4)$$

A loss tangent $tan\delta$ is expressed by Equation (5). That is, the loss tangent $tan\delta$ is a value obtained by dividing the loss elastic modulus $G''(\omega)$ by the storage elastic modulus $G'(\omega)$.
[Math. 5]

$$tan\delta = \frac{G''(\omega)}{G'(\omega)} \qquad \cdots \quad (5)$$

In the present embodiment, the dynamic viscoelastic properties of the polymer composite material include the storage elastic modulus $G'(\omega)$, the loss elastic modulus $G''(\omega)$, and the loss tangent $tan\delta$.

3. Configuration of simulation device 1

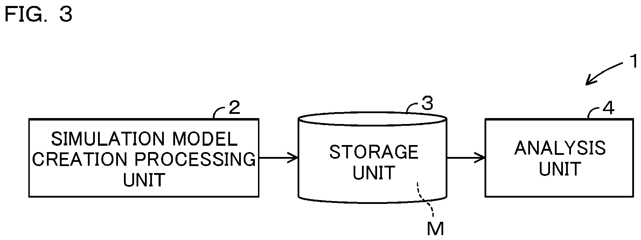

A configuration of a simulation device 1 will be described with reference to Fig. 3. The simulation device 1 sets the polymer composite material illustrated in Fig. 1 as the simulation target P. The simulation device 1 is a device for analyzing a dynamic viscoelastic property in a case where a vibration strain is applied to a polymer composite material in which a filler is non-uniformly distributed in a matrix polymer as the target P. The simulation device 1 analyzes the storage elastic modulus $G'(\omega)$, the loss elastic modulus $G''(\omega)$, and the loss tangent $tan\delta$ as the dynamic viscoelastic property of the polymer composite material.
As illustrated in Fig. 3, the simulation device 1 includes a simulation model creation processing unit 2, a storage unit 3, and an analysis unit 4. The simulation model creation processing unit 2 creates a model M of the polymer composite material which is the target P illustrated in Fig. 1. The storage unit 3 stores the created simulation model M. The analysis unit 4 analyzes the storage elastic modulus G', the loss elastic modulus G", and the loss tangent $tan\delta$ of the polymer composite material by solving a dynamic equilibrium equation in a case where a vibration strain is applied to the simulation model M.

4. Processing of simulation model creation processing unit 2

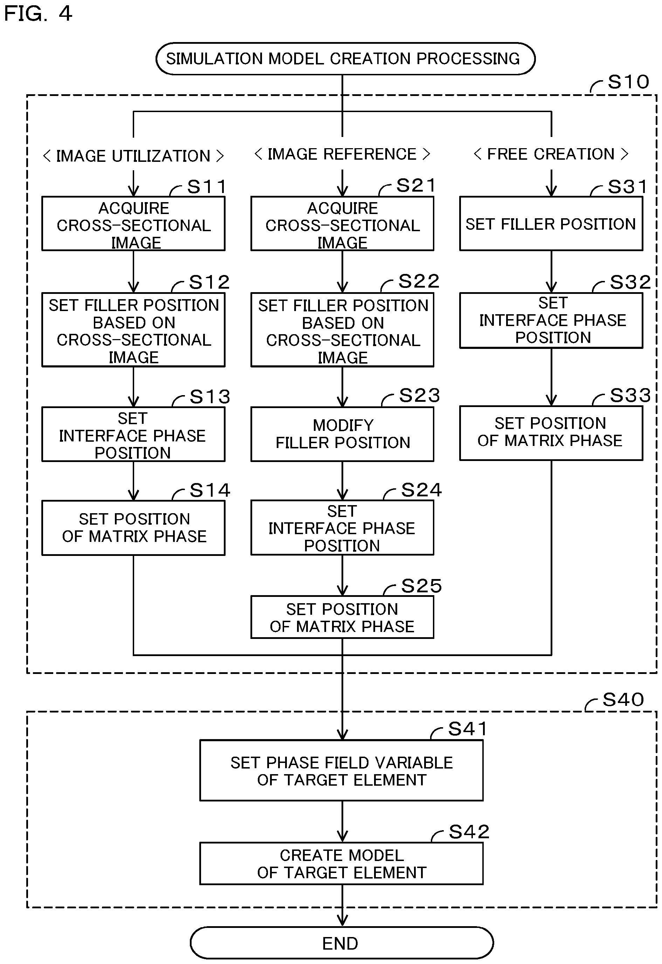

Processing of the simulation model creation processing unit 2 will be described with reference to Figs. 4 to 10. As shown in Fig. 4, the simulation model creation processing unit 2 executes an element position setting process S10 of setting an element position of the simulation model M, and then executes a parameter setting process S40 of setting a parameter of each element of the simulation model M.

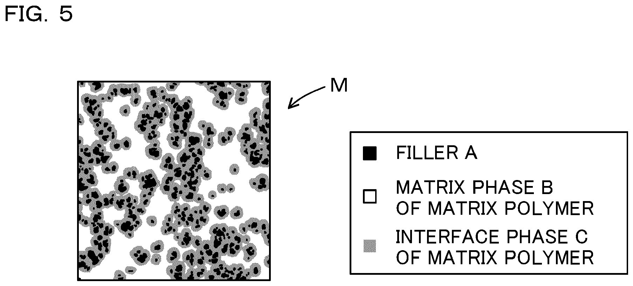

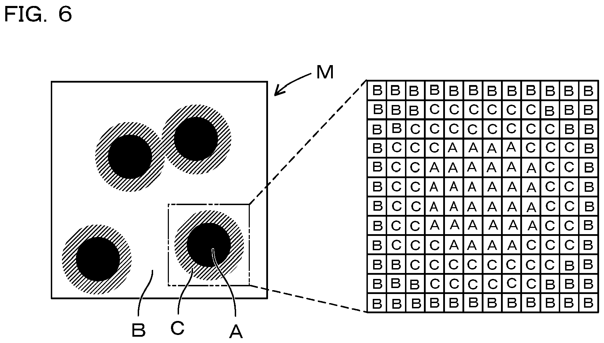

The element position setting process S10 sets a simulation model M having a definition region of the meso scale, that is, the definition region having one side of 10 nm to 100 $\mu$m as illustrated in Figs. 5 and 6. When the simulation model M is set as a model having a meso scale definition region, it is possible to evaluate the dynamic viscoelastic property, which is the object described above. In particular, the simulation model M is used as a model for grasping the effect caused by the dispersion state of the filler A on the viscoelastic property of the polymer composite material.

As illustrated in Figs. 5 and 6, the simulation model M is a model formed by a model of the filler A, a model of a matrix phase B of the matrix polymer, and a model of an interface phase C of the matrix polymer. That is, the element position setting process S10 is a process of setting a position of the filler A, a position of the matrix phase B, and a position of the interface phase C in the definition region of the simulation model M.

In the present embodiment, the filler A is, for example, carbon black. However, as the filler A, in addition to carbon black, a filler for exhibiting various functions can be applied. The matrix polymer is a rubber or an elastomer, and in the present embodiment, for example, styrene-butadiene rubber (SBR) or the like is applied.

As illustrated in Figs. 5 and 6, the simulation model M includes a plurality of models of fillers A arranged in a non-uniformly dispersed manner. A model of the filler A is formed by aggregation of nanofillers. Thus, individual models of the fillers A are different in shape and size.

The model of the matrix phase B of the matrix polymer is located in the region present as a matrix polymer monolayer. Thus, the model of the matrix phase B of the matrix polymer has a property of the matrix polymer monolayer. The model of the matrix phase B is located at a position away from the model of the filler A.

The model of the interface phase C of the matrix polymer is located between the model of the matrix phase B of the matrix polymer and the model of the filler A. In particular, the model of the interface phase C is located on the surface of the model of the filler A. Thus, the model of the interface phase C is represented by the thickness from the surface of the model of the filler A. That is, the position of the model of the interface phase C is set from the surface of the model of the filler A based on the thickness information of the interface phase C set in advance. Here, the thickness of the model of the interface phase C can be set in advance, and can be appropriately set depending on the material of the matrix polymer, the material of the filler, and the like.

Returning to Fig. 4, detailed processing of the element position setting process S 10 will be described. The element position setting process S10 can set the element position of the simulation model M by, for example, a method selected from three types. The element position setting process S10 can perform setting by any of an image utilization method, an image reference method, and a free creation method.

In the image utilization method as one of the element position setting process S 10, first, a cross-sectional image of the polymer composite material of the simulation target P as illustrated in Fig. 1 is acquired (S 11). The cross-sectional image is represented by the filler A and the matrix polymer. Subsequently, the position of the filler A is set based on the cross-sectional image (S12). For example, the cross-sectional image can be binarized to specify the position of the filler A.

Subsequently, the position of the interface phase C is set (S13). Based on the thickness information of the interface phase C set in advance, the position of the interface phase C is set to a region of a thickness corresponding to the thickness information from the surface of the filler A. Subsequently, the remaining region of the matrix polymer is set as the position of the matrix phase B (S14). In this way, the position of the filler A, the position of the matrix phase B, and the position of the interface phase C are set in the definition region of the simulation model M.

In the image reference method as one of the element position setting process S10, first, a cross-sectional image of the polymer composite material of the simulation target P as illustrated in Fig. 1 is acquired (S21). The cross-sectional image is represented by the filler A and the matrix polymer. Subsequently, the position of the filler A is set based on the cross-sectional image (S22). For example, the cross-sectional image can be binarized to specify the position of the filler A. Subsequently, the position of the filler A is corrected by an operation of an operator (S23).

Subsequently, the position of the interface phase C is set (S24). Based on the thickness information of the interface phase C set in advance, the position of the interface phase C is set to a region of a thickness corresponding to the thickness information from the surface of the filler A. Subsequently, the remaining region of the matrix polymer is set as the position of the matrix phase B (S25). In this way, the position of the filler A, the position of the matrix phase B, and the position of the interface phase C are set in the definition region of the simulation model M.

In the image reference method as one of the element position setting process S10, first, the position of the filler A is set in the definition region of the simulation model M by the operation of the operator (S31). Subsequently, the position of the interface phase C is set (S32). Based on the thickness information of the interface phase C set in advance, the position of the interface phase C is set to a region of a thickness corresponding to the thickness information from the surface of the filler A. Subsequently, the remaining region of the matrix polymer is set as the position of the matrix phase B (S33). In this way, the position of the filler A, the position of the matrix phase B, and the position of the interface phase C are set in the definition region of the simulation model M.

In the parameter setting process S40, first, a phase field variable of the model of the interface phase C is set as a target element (S41). Subsequently, a model of the filler A, a model of the matrix phase B, and a model of the interface phase C as target elements constituting the simulation model M are created (S42). In this manner, the simulation model M is created.

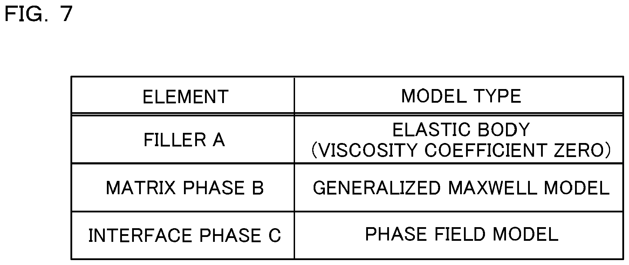

Here, in the present embodiment, respective model types of the elements A, B, and C of the simulation model M are set as shown in Fig. 7. As shown in Fig. 7, the model of the filler A is defined as an elastic body, and has a viscosity coefficient of zero. The model of the filler A defined as an elastic body is a model having a constant elastic modulus. For example, the model of filler A has an elastic modulus of 70 GPa.

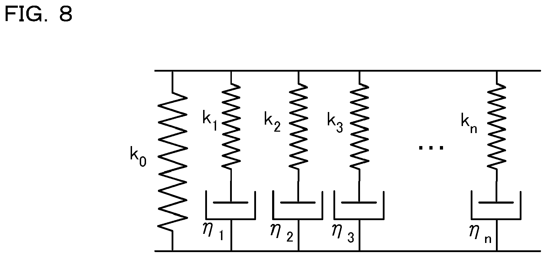

As shown in Fig. 7, the model of the matrix phase B of the matrix polymer is defined as a generalized Maxwell model. In the generalized Maxwell model, as illustrated in Fig. 8, a plurality of series units in each of which a spring and a dash pod are arranged in series are arranged in parallel. The spring represents an elastic behavior and the dash pod represents a viscous behavior.

A storage elastic modulus $G'_r(\omega)$ in the generalized Maxwell model is expressed by Equation (6). A loss elastic modulus $G''_r(\omega)$ in the generalized Maxwell model is expressed by Equation (7). From Equations (6) and (7), the storage elastic modulus $G'_r(\omega)$ and the loss elastic modulus $G''_r(\omega)$ in the model of the matrix phase B are defined as different values depending on the angular frequency $\omega$ of the vibration strain.

[Math. 6]

$$G'_r(\omega) = k_0 + \sum_{i=1}^{n} k_i \frac{\omega^2 \tau_i^2}{1 + \omega^2 \tau_i^2} \qquad \cdots (6)$$

$G'_r(\omega)$ : Storage elastic modulus of matrix phase of matrix polymer

$\omega$ : Angular frequency

$k_i$ : Elastic modulus of i-th Maxwell model

$\eta_i$ : Viscosity coefficient of i-th Maxwell model

$\tau_i$ : Relaxation time ($\tau_i = \eta_i / k_i$)[Math. 7]

$$G''_r(\omega) = \sum_{i=1}^{n} k_i \frac{\omega \tau_i}{1 + \omega^2 \tau_i^2} \qquad \cdots (7)$$

$G''_r(\omega)$ : Loss elastic modulus of matrix phase of matrix polymer

In the present embodiment, for example, a storage elastic modulus $G''_g$ in a glassy state of the matrix polymer as SBR is 2 to 3.5 GPa, and a loss elastic modulus $G''_g$ in the glassy state of the matrix polymer as SBR is 0 Pa. However, these values can also be freely set.

As shown in Fig. 7, the model of the interface phase C of the matrix polymer is defined as a model (phase field model) represented using a phase field variable x as shown in Equation (8). According to Equation (8), in the model of the interface phase C, a complex elastic modulus $G^*_1$ is defined using the phase field variable x. The phase field variable x is a variable for setting a ratio between a complex elastic modulus $G^*_r$ of the matrix polymer in the rubbery state and a complex elastic modulus $G^*_g$ of the matrix polymer in the glassy state.

As shown in Expression (8), the model of the interface phase C is defined as a different value depending on the angular frequency $\omega$ of the vibration strain. The phase field variable x is defined as a value independent of the angular frequency ω of the vibration strain. However, the phase field variable x can also be defined as a different value depending on the angular frequency ω of the vibration strain.

[Math. 8]

$$G_i^*(\omega) = G_g^* + x\left(G_r^*(\omega) - G_g^*\right) \qquad \cdots (8)$$

$G_i^*(\omega)$ : Complex elastic modulus of interface phase of matrix polymer

$G_g^*$ : Complex elastic modulus of matrix polymer in glassy state

$G_r^*(\omega)$ : Complex elastic modulus of matrix polymer in rubbery state (Generalized Maxwell Model)

Here, as shown in Equation (9), the complex elastic modulus $G_g^*$ of the matrix polymer in the glassy state can be represented by the storage elastic modulus $G_g'$ of the matrix polymer in the glassy state and the loss elastic modulus $G_g''$ of the matrix polymer in the glassy state. The storage elastic modulus $G_g''$ and the loss elastic modulus $G_g''$ in the glassy state are defined as values independent of the angular frequency ω of the vibration strain. Accordingly, the complex elastic modulus $G_g^*$ in the glassy state is defined as a value independent of the angular frequency ω of the vibration strain.

[Math. 9]

$$G_g^* = G_g' + iG_g'' \qquad \cdots (9)$$

$G_g'$ : Storage elastic modulus of matrix polymer in glassy state

$G_g''$ : Loss elastic modulus of matrix polymer in glassy state

Furthermore, as shown in Equation (10), a complex elastic modulus $G_r^*(\omega)$ of the matrix polymer in the rubbery state can be represented by the storage elastic modulus $G_r'(\omega)$ of the matrix polymer in the rubbery state and the loss elastic modulus $G_r''(\omega)$ of the matrix polymer in the rubbery state. The storage elastic modulus $G_r'(\omega)$ and the loss elastic modulus $G_r''(\omega)$ in the rubbery state are defined as values that are deferent depending on the angular frequency ω of the vibration strain. Accordingly, the complex elastic modulus $G_r^*(\omega)$ in the rubbery state is defined as a different value depending on the angular frequency ω of the vibration strain.

The storage elastic modulus $G_r'(\omega)$ of the matrix polymer in the rubbery state is equal to the storage elastic modulus $G_r'(\omega)$ in the matrix phase B of the matrix polymer described above. Furthermore, the loss elastic modulus $G_r''(\omega)$ of the matrix polymer in the rubbery state is equal to the loss elastic modulus $G_r''(\omega)$ in the matrix phase B of the matrix polymer described above.

[Math. 10]

$$G_r^*(\omega) = G_r'(\omega) + iG_r''(\omega) \qquad \cdots (10)$$

$G_r'(\omega)$ : Storage elastic modulus of matrix polymer in rubbery state

$G_r''(\omega)$ : Loss elastic modulus of matrix polymer in rubbery state

When Equations (9) and (10) are substituted into Equation (8), the complex elastic modulus $G_i^*$ of the model of the interface phase C is expressed as Equation (11). When Equation (11) is developed to separate a real part and an imaginary part, the storage elastic modulus $G_i'$ of the model of the interface phase C corresponding to the real part is represented as Equation (12), and the loss elastic modulus $G_i''$ of the model of the interface phase C corresponding to the imaginary part is represented as Expression (13). From Equations (12) and (13), the storage elastic modulus $G'_i$ and the loss elastic modulus $G''_i$ of the model of the interface phase C are defined as different values depending on the angular frequency $\omega$ of the vibration strain.

[Math. 11]

$$G_i^*(\omega) = G_g' + x\left(G_r'(\omega) - G_g'\right) + i\left[G_g'' + x\left(G_r''(\omega) - G_g''\right)\right]$$

$$\cdots \quad (1\,1)$$

[Math. 12]

$$G_i'(\omega) = G_g' + x\left(G_r'(\omega) - G_g'\right) \qquad \cdots \quad (1\,2)$$

[Math. 13]

$$G_i''(\omega) = G_g'' + x\left(G_r''(\omega) - G_g''\right) \qquad \cdots \quad (1\,3)$$

From Equations (12) and (13), the model of the interface phase C is a model in which the storage elastic modulus $G'_i$ and the loss elastic modulus $G''_i$ in the interface phase C are defined using the phase field variable x. The phase field variable x takes a value in a range of 0 to 1. When the phase field variable x in the interface phase C is 1, the model of the interface phase C matches the rubbery state of the matrix polymer. That is, when the phase field variable x in the interface phase C is 1, behavior similar to that of the model of the matrix phase B is exhibited. On the other hand, when the phase field variable x in the interface phase C is 0, the model of the interface phase C matches the glassy state of the matrix polymer.

It is possible to freely set the storage elastic modulus $G'_i$ and the loss elastic modulus $G''_i$ in the interface phase C by varying the phase field variable x. That is, it is possible to freely set whether the interface phase C is brought into a state close to the rubbery state or a state close to the glassy state by varying the phase field variable x. Thus, setting of the storage elastic modulus $G'_i$ and the loss elastic modulus $G''_i$ in the interface phase C has a high degree of freedom and is easy.

Here, the model of the interface phase C can be a model in which the phase field variable x is defined as one value. Alternatively, In the model of the interface phase C, the phase field variable x can be defined as different values at the position on the matrix phase B side and at the position on the filler A side. In a case where the phase field variable x is set to different values, the phase field variable x can be varied stepwise or continuously.

Furthermore, in the model of the interface phase C, the phase field variable x can be set to have different values in the storage elastic modulus $G'_i$ and the loss elastic modulus $G''_i$ in the interface phase C. Accordingly, in the interface phase C, the phase field variable x of the storage elastic modulus $G'_i$ and the phase field variable x of the loss elastic modulus $G''_i$ can be defined as different values. Of course, in the interface phase C, it is also possible to define the phase field variable x of the storage elastic modulus $G'_i$ and the phase field variable x of the loss elastic modulus $G''_i$ as an identical value.

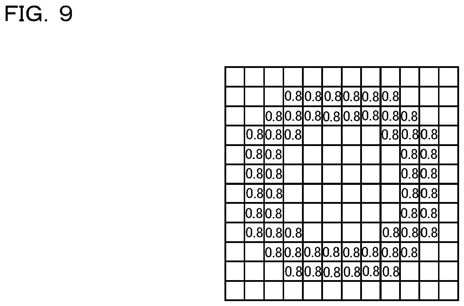

In the present embodiment, the model of the interface phase C is a model having a region in which the phase field variable x is set so as to include both the rubbery state and the glassy state of the matrix polymer. As shown in Fig. 9, the phase field variable x is applied only to the model of the interface phase C, and is set to one value of 0.8, for example. That is, as a region in which the phase field variable x is set to include both the rubbery state and the glassy state of the matrix polymer, the phase field variables x at all positions in the region of the interface phase C have the same value. Furthermore, in the interface phase C, the phase field variable x of the storage elastic modulus $G'_i$ and the phase field variable x of the loss elastic modulus $G''_i$ are set to the same value.

As described above, for example, the storage elastic modulus $G''_g$ in the glassy state of the matrix polymer as SBR is 2 to 3.5 GPa, and the loss elastic modulus $G''_g$ in the glassy state of the matrix polymer as SBR is 0 Pa. However, these values can also be freely set.

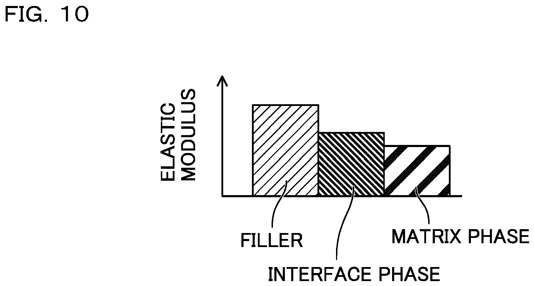

In a case where the phase field variable x is one value, the elastic moduli of the filler A, the matrix phase B, and the interface phase C have a relationship as shown in Fig. 10. That is, the elastic modulus of the filler A becomes the largest value, the elastic modulus of the matrix phase B becomes the smallest value, and the elastic modulus of the interface phase C becomes an elastic modulus between that of the filler A and that of the matrix phase B.

As described above, the simulation model M is created. That is, the position of the filler A, the model of the filler A, the position of the matrix phase B, the model of the matrix phase B, the position of the interface phase C, and the model of the interface phase C, which constitute the simulation model M, are created. Here, the model of the filler A is a model that is defined as an elastic body and has a viscosity coefficient of zero. The model of the matrix phase B is defined as a generalized Maxwell model. Furthermore, the model of the matrix phase B is set as a different value depending on the angular frequency $\omega$ of the vibration strain. In the model of the matrix phase B, the storage elastic modulus $G'_g$ in the glassy state of the matrix polymer as SBR is 2 to 3.5 GPa, and the loss elastic modulus $G''_g$ in the glassy state of the matrix polymer as SBR is 0 Pa.

The model of the interface phase C is defined using the phase field variable x, and is further set to a different value depending on the angular frequency $\omega$ of the vibration strain. Each of the storage elastic modulus $G'_i$ and the loss elastic modulus $G''_i$ constituting the model of the interface phase C is defined using the phase field variable x. The created simulation model M is stored in the storage unit 3.

5. Processing of analysis unit 4

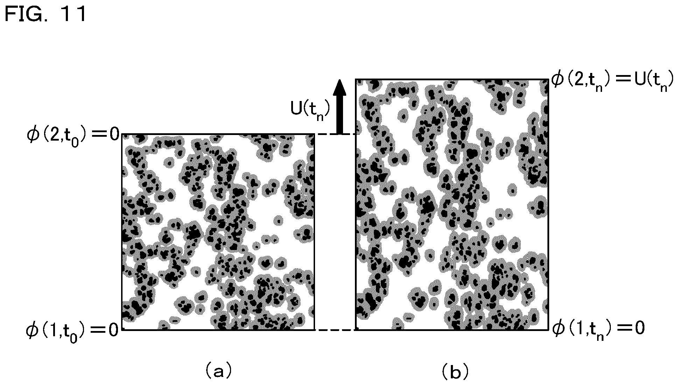

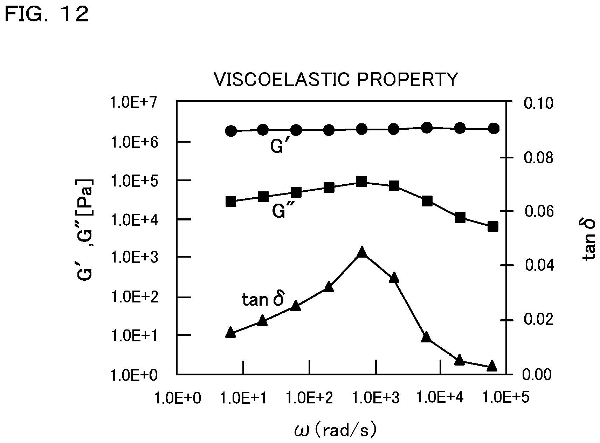

Processing of the analysis unit 4 will be described with reference to Figs. 11 and 12. The analysis unit 4 analyzes the storage elastic modulus, the loss elastic modulus, and the loss tangent tan$\delta$ of the polymer composite material by solving a dynamic equilibrium equation in a case where a vibration strain U(t) is applied to the simulation model M stored in the storage unit 3.

The vibration strain U(t) is expressed by Equation (14).

[Math. 14]

$$U(t) = U_0 \, exp(i\omega t) \qquad \cdots (14)$$

$U(t)$ : Externally applied strain
$U_0$ : Externally applied strain amplitude

A boundary condition of the vibration strain U(t) will be described with reference to Fig. 11. Fig. 11(a) illustrates a state in which the vibration strain U(t) is not applied, and Fig. 11(b) illustrates a state at a certain moment $t_n$ at which the vibration strain U(t) is applied. In Figs. 11(a) and 11(b), a lower edge is set as a reference edge, and an upper edge is set as a movable edge. That is, in a case where the vibration strain U(t) is applied, the movable edge is displaced without displacing the reference edge.

As illustrated in Fig. 11(a), in a state where the vibration strain U(t) is not applied, the boundary condition of the reference edge is as shown in Equation (15), and the boundary condition of the movable edge is as shown in Equation (16). In addition, as illustrated in Fig. 11(b), in a state where the vibration strain U(t) is not applied, the boundary condition of the reference edge is as shown in Equation (17), and the boundary condition of the movable edge is as shown in Equation (18).

[Math. 15]

$$\phi(1, t_0) = 0 \qquad \cdots (15)$$

$\phi(1, t_0)$ : Displacement of reference edge at initial time to

[Math. 16]

$$\phi(2, t_0) = 0 \qquad \cdots (16)$$

$\phi(2, t_0)$ : Displacement of movable edge at initial time $t_0$

[Math. 17]

$$\phi(1, t_n) = 0 \qquad \cdots (17)$$

$\phi(1, t_n)$ :　　Displacement of reference edge at time $t_n$

[Math. 18]

$$\phi(2, t_n) = U(t_n) \qquad \cdots (18)$$

$\phi(2, t_n)$ :　　Displacement of movable edge at time $t_n$

In the simulation model M, an internal strain field is expressed as Equation (19). Furthermore, in the simulation model M, when expressed as an internal complex displacement field, the internal strain field is as Equation (20).

[Math. 19]

$$E(r,t) = E_0(r)\, exp(i\omega t) \qquad \cdots (19)$$

$E(r,t)$ :　　Strain in simulation model at time t
$E_0(r)$ :　　Strain amplitude in simulation model
$r$ :　　Position in simulation model

[Math. 20]

$$\phi(r,t) = \phi_0(r)\, exp(i\omega t) = [\phi_{R0}(r) + i\phi_{I0}(r)]\, exp(i\omega t)$$

$$\cdots (20)$$

$\phi(r,t)$　　: Displacement at time t
$\phi_0(r)$　　: Amplitude of displacement
$\phi_{R0}(r)$　　: Amplitude of real part of displacement
$\phi_{I0}(r)$　　: Amplitude of imaginary part of displacement
$r$　　: Position in simulation model

In the microstrain theory, a strain E(r, t) in the simulation model M defined by Equation (19) and a displacement φ(r, t) defined by Equation (20) have a relationship of Equation (21).

[Math. 21]

$$E(r,t) = \nabla\phi(r,t) \qquad \cdots (21)$$

In addition, in a case where a complex elastic modulus C(r), a storage elastic modulus C'(r), and a loss elastic modulus C"(r) of the polymer composite material are defined as a function of the position r, the complex elastic modulus C(r), the storage elastic modulus C'(r), and the loss elastic modulus C"(r) are expressed by Equation (22).

[Math. 22]

$$C(r) = C'(r) + iC''(r) \qquad \cdots (22)$$

$C(r)$ :　　Complex elastic modulus of polymer composite material (function of position only)
$C'(r)$ :　　Storage elastic modulus of polymer composite material (function of position only)
$C''(r)$ :　　Loss elastic modulus of polymer composite material (function of position only)

Then, the analysis unit 4 solves the dynamic equilibrium equation represented by Equation (23) by numerical analysis. When Equation (23) is decomposed into a real part and an imaginary part, Equations (24) and (25) are obtained. Thus, the analysis unit 4 can solve Equations (24) and (25) by numerical analysis.

[Math. 23]

$$\nabla \cdot \{C(r)E\} = 0 \qquad \cdots (23)$$

[Math. 24]

$$\nabla \cdot [C_{Re}(r)\nabla \phi_{Re0}(r)] - \nabla \cdot [C_{Im}(r)\nabla \phi_{Im0}(r)] = 0 \qquad \cdots (24)$$

[Math. 25]

$$\nabla \cdot [C_{Im}(r)\nabla \phi_{Re0}(r)] + \nabla \cdot [C_{Re}(r)\nabla \phi_{Im0}(r)] = 0 \qquad \cdots (25)$$

The analysis unit 4 can apply various numerical analysis methods such as an iterative method and a direct method. For example, the analysis unit 4 can apply a SOR method (successive over-relaxation method), a Jacobian method, a Gauss-Seidel method as the iterative method, a Gauss elimination method, a LU decomposition method as the direct method, or the like. As a result of numerical analysis, the analysis unit 4 can obtain the storage elastic modulus G', the loss elastic modulus G", and the loss tangent $\tan\delta$ of the polymer composite material.

The storage elastic modulus G', the loss elastic modulus G", and the loss tangent $\tan\delta$ of the polymer composite material obtained by the analysis unit 4 are shown in Fig. 12. As shown in Fig. 12, the storage elastic modulus G', the loss elastic modulus G", and the loss tangent $\tan\delta$ of the polymer composite material are obtained as values depending on the angular frequency $\omega$ of the vibration strain U(t).

As described above, according to the simulation device 1 described above, the model of the interface phase C is a model defined using the phase field variable x. When the phase field variable x is varied, the properties of the model of the interface phase C can be freely set. Thus, the model of the interface phase C has a high degree of freedom and is easily set. As a result, the viscoelastic property of the polymer composite material can be simulated with high accuracy.

(Second Embodiment)

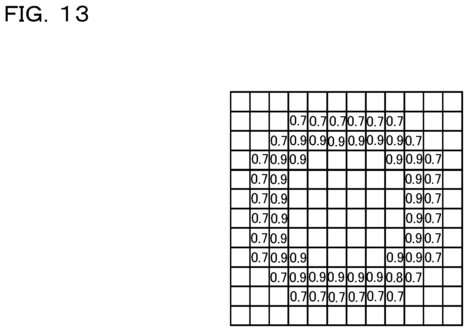

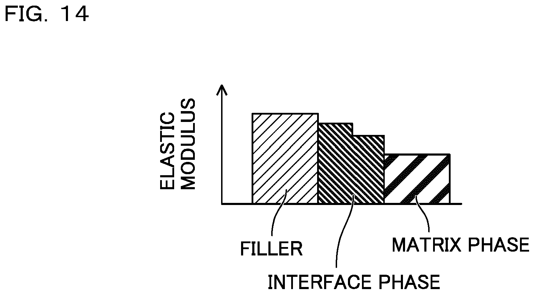

A simulation device 1 according to a second embodiment will be described with reference to Figs. 13 and 14. In the simulation device 1 of the present embodiment, as illustrated in Fig. 13, a phase field variable x in a model of an interface phase C is defined as a value varies depending on the position of the interface phase C.

The model of the interface phase C is a model having a region in which the phase field variable x is set so as to include both a rubbery state and ta glassy state of the matrix polymer. As shown in Fig. 13, the model of the interface phase C is a model in which as a region in which the phase field variable x is set to include both the rubbery state and the glassy state of the matrix polymer, the phase field variable x is defined to have different values at a position on the matrix phase B side and a position on the filler A side. For example, in the model of the interface phase C, the phase field variable x on the filler A side is defined as 0.9, and the phase field variable x on the matrix phase B side is defined as 0.7. That is, the model of the interface phase C is a model in which the phase field variable x is defined as a value that varies stepwise from the matrix phase B side toward the filler A side.

In a case where the phase field variable x is defined as shown in Fig. 13, the elastic moduli of the filler A, the matrix phase B, and the interface phase C have a relationship as illustrated in Fig. 14. That is, the elastic modulus of the filler A becomes the largest value, the elastic modulus of the matrix phase B becomes the smallest value, and the elastic modulus of the interface phase C becomes a two-step elastic modulus between the filler A and the matrix phase B. Such setting can also be realized by setting the phase field variable x, and is very easy.

(Third Embodiment)

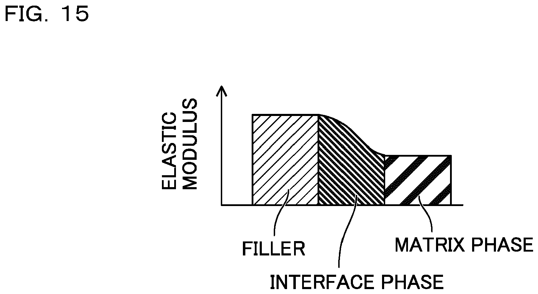

A simulation device 1 according to a third embodiment will be described with reference to Fig. 15. In the simulation device 1 of the present embodiment, a phase field variable x in a model of an interface phase C is defined as a value different depending on the position of the interface phase C. Furthermore, the model of the interface phase C is a model in which a phase field variable x is defined as a value varying continuously from the matrix phase B side toward the filler A side. For example, the phase field variable x continuously varies as $0.1 \rightarrow 0.2 \rightarrow 0.3 \rightarrow 0.4 \rightarrow 0.5 \rightarrow 0.6 \rightarrow 0.7 \rightarrow 0.8 \rightarrow 0.9$ from the matrix phase B side toward the filler A side.

In this case, the elastic moduli of the filler A, the matrix phase B, and the interface phase C can have a relationship as shown in Fig. 15. That is, the elastic modulus of the filler A becomes the largest value, the elastic modulus of the matrix phase B becomes the smallest value, and the elastic modulus of the interface phase C becomes an elastic modulus varying continuously between the filler A and the matrix phase B. Such setting can also be realized by setting the phase field variable x, and is very easy.

(Fourth Embodiment)

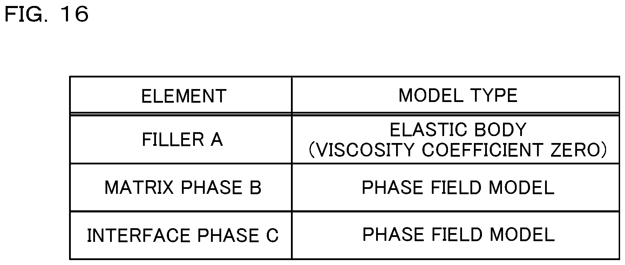

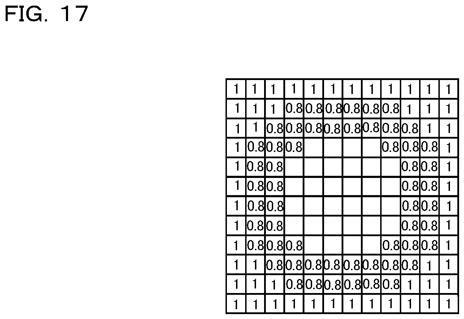

A simulation device 1 according to a fourth embodiment will be described with reference to Figs. 16 and 17. In the simulation device 1 of the present embodiment, model types of respective elements A, B, and C of the simulation model M of the polymer composite material are set as shown in Fig. 16. As shown in Fig. 16, the model of the filler A is defined as an elastic body, and has a viscosity coefficient of zero.

The model of the matrix phase B is a model in which a storage elastic modulus $G'_r(\omega)$ and a loss elastic modulus $G''_r(\omega)$ in the matrix phase B are defined using a phase field variable x for setting a ratio between a rubbery state and a glassy state of the matrix polymer. The model of the matrix phase B is set in the same manner as the model of the interface phase C in the first embodiment. However, the phase field variable x is set to a value different from that of the interface phase C.

For example, in Fig. 17, the phase field variable x in the model of the matrix phase B is set to 1. However, the phase field variable x in the model of the matrix phase B can be set to a value other than 1, and can be set to a different value depending on the position of the matrix phase B.

As described above, the degree of freedom of the simulation model M of the polymer composite material is increased by adopting a model using the phase field variable x not only for the interface phase C but also for the matrix phase B. Furthermore, the setting is easy despite the high degree of freedom. Thus, more actual simulation of a polymer composite material can be realized, and desired results can be obtained.

**Claims**

1.  A device (1) for simulating a viscoelastic property of a polymer composite material in which a filler (A) is dispersed in a matrix polymer (B, C), the device comprising:

    a storage unit (3) that stores a simulation model (M) formed by a model of a matrix phase (B) of the matrix polymer, a model of the filler, and a model of an interface phase (C) of the matrix polymer located between the matrix phase of the matrix polymer and the filler; and
    an analysis unit (4) that analyzes a storage elastic modulus and a loss elastic modulus of the polymer composite material by solving a dynamic equilibrium equation in a case where a vibration strain (U(t)) is applied to the simulation model, wherein
    the model of the interface phase is a model in which a storage elastic modulus and a loss elastic modulus in the interface phase are defined using a phase field variable (x) for setting a ratio between a rubbery state and a glassy state of the matrix polymer.

2.  The device for simulating a viscoelastic property of a polymer composite material according to claim 1, wherein the model of the interface phase is a model in which the phase field variable is defined as different values at a position on the matrix phase side and at a position on the filler side.

3.  The device for simulating a viscoelastic property of a polymer composite material according to claim 2, wherein the model of the interface phase is a model in which the phase field variable is defined as a value continuously varying from the matrix phase side to the filler side.

4.  The device for simulating a viscoelastic property of a polymer composite material according to claim 1, wherein the model of the interface phase is a model in which the phase field variable is defined as one value.

5.  The device for simulating a viscoelastic property of a polymer composite material according to claim 4, wherein

the model of the interface phase is defined as a value that varies depending on an angular frequency of the vibration strain,

in the model of the interface phase, at least the storage elastic modulus and the loss elastic modulus in the rubbery state of the matrix polymer are defined as values that vary depending on the angular frequency of the vibration strain, and

in the model of the interface phase, the phase field variable is defined as a value independent of the angular frequency of the vibration strain.

6.  The device for simulating a viscoelastic property of a polymer composite material according to any one of claims 1 to 5, wherein the model of the interface phase is configured such that the phase field variable is settable to different values in the storage elastic modulus and the loss elastic modulus of the interface phase, and the phase field variable of the storage elastic modulus of the interface phase and the phase field variable of the loss elastic modulus of the interface phase are defined to be an identical value.

7.  The device for simulating a viscoelastic property of a polymer composite material according to any one of claims 1 to 6, wherein the storage elastic modulus and the loss elastic modulus in the rubbery state of the matrix polymer are equal to the storage elastic modulus and the loss elastic modulus of the matrix phase.

8.  The device for simulating a viscoelastic property of a polymer composite material according to any one of claims 1 to 7, wherein the model of the matrix phase is a generalized Maxwell model.

9.  The device for simulating a viscoelastic property of a polymer composite material according to any one of claims 1 to 7, wherein the model of the matrix phase is a model in which the storage elastic modulus and the loss elastic modulus in the matrix phase are defined using the phase field variable for setting the ratio between the rubbery state and the glassy state of the matrix polymer.

10. The device for simulating a viscoelastic property of a polymer composite material according to any one of claims 1 to 9, wherein the model of the filler is a model defined as an elastic body and having a viscosity coefficient of 0.

11. The device for simulating a viscoelastic property of a polymer composite material according to any one of claims 1 to 10, wherein the simulation model is a model in which the model of the filler is non-uniformly arranged.

12. The device for simulating a viscoelastic property of a polymer composite material according to any one of claims 1 to 11, the device further comprising

a simulation model creation processing unit (2) configured to be able to create the simulation model, wherein the simulation model creation processing unit is configured to be able to set a position of the matrix phase, a position of the filler, and a position of the interface phase in a definition region of the simulation model.

13. The device for simulating a viscoelastic property of a polymer composite material according to claim 12, wherein the simulation model creation processing unit is configured to acquire a cross-sectional image of the polymer composite material, and be able to set the position of the matrix phase, the position of the filler, and the position of the interface phase based on the cross-sectional image.

14. The device for simulating a viscoelastic property of a polymer composite material according to claim 12 or 13, wherein the position of the interface phase is set based on preset thickness information.

15. The device for simulating a viscoelastic property of a polymer composite material according to any one of claims 1 to 14, wherein the simulation model is a model having a meso scale definition region, and is a model for grasping an effect caused by a dispersion state of the filler on the viscoelastic property of the polymer composite material.

FIG. 1

P

| | |
|---|---|
| ■ | FILLER |
| □ | MATRIX POLYMER |

FIG. 2

FIG. 3

```
                                                              ⟍1

           ┌─────────────────┐2  ┌──────────┐3  ┌──────────┐4
           │ SIMULATION MODEL │   │          │   │          │
           │CREATION PROCESSING│  │ STORAGE  │   │ ANALYSIS │
           │      UNIT        │──▶│   UNIT   │──▶│   UNIT   │
           └─────────────────┘   └──────────┘   └──────────┘
                                        ⌇
                                        M
```

FIG. 4

```
           ┌─────────────────────────────────────────────┐
           │   SIMULATION MODEL CREATION PROCESSING      │
           └─────────────────────────────────────────────┘
```

┌─ S10

**< IMAGE UTILIZATION >**

┌─ S11
ACQUIRE
CROSS-SECTIONAL
IMAGE

┌─ S12
SET FILLER POSITION
BASED ON
CROSS-SECTIONAL
IMAGE

┌─ S13
SET
INTERFACE PHASE
POSITION

┌─ S14
SET POSITION
OF MATRIX PHASE

**< IMAGE REFERENCE >**

┌─ S21
ACQUIRE
CROSS-SECTIONAL
IMAGE

┌─ S22
SET FILLER POSITION
BASED ON
CROSS-SECTIONAL
IMAGE

┌─ S23
MODIFY
FILLER POSITION

┌─ S24
SET
INTERFACE PHASE
POSITION

┌─ S25
SET POSITION
OF MATRIX PHASE

**< FREE CREATION >**

┌─ S31
SET FILLER POSITION

┌─ S32
SET
INTERFACE PHASE
POSITION

┌─ S33
SET POSITION
OF MATRIX PHASE

┌─ S40

┌─ S41
SET PHASE FIELD VARIABLE
OF TARGET ELEMENT

┌─ S42
CREATE MODEL
OF TARGET ELEMENT

```
           ┌─────────┐
           │   END   │
           └─────────┘
```

FIG. 5

M

| | |
|---|---|
| ■ | FILLER A |
| □ | MATRIX PHASE B OF MATRIX POLYMER |
| ▦ | INTERFACE PHASE C OF MATRIX POLYMER |

FIG. 6

FIG. 7

| ELEMENT | MODEL TYPE |
|---|---|
| FILLER A | ELASTIC BODY (VISCOSITY COEFFICIENT ZERO) |
| MATRIX PHASE B | GENERALIZED MAXWELL MODEL |
| INTERFACE PHASE C | PHASE FIELD MODEL |

FIG. 8

FIG. 9

| | | | | | | | | | | | |
|---|---|---|---|---|---|---|---|---|---|---|---|
| | | | 0.8 | 0.8 | 0.8 | 0.8 | 0.8 | 0.8 | | | |
| | | 0.8 | 0.8 | 0.8 | 0.8 | 0.8 | 0.8 | 0.8 | 0.8 | | |
| | 0.8 | 0.8 | 0.8 | | | | | 0.8 | 0.8 | 0.8 | |
| | 0.8 | 0.8 | | | | | | | 0.8 | 0.8 | |
| | 0.8 | 0.8 | | | | | | | 0.8 | 0.8 | |
| | 0.8 | 0.8 | | | | | | | 0.8 | 0.8 | |
| | 0.8 | 0.8 | | | | | | | 0.8 | 0.8 | |
| | 0.8 | 0.8 | 0.8 | | | | | 0.8 | 0.8 | 0.8 | |
| | | 0.8 | 0.8 | 0.8 | 0.8 | 0.8 | 0.8 | 0.8 | 0.8 | | |
| | | | 0.8 | 0.8 | 0.8 | 0.8 | 0.8 | 0.8 | | | |
| | | | | | | | | | | | |

FIG. 10

FIG. 11

$\phi(2,t_0)=0$

$\phi(1,t_0)=0$

$U(t_n)$

$\phi(2,t_n)=U(t_n)$

$\phi(1,t_n)=0$

(a)

(b)

FIG. 12

FIG. 13

| | | | | | | | | | | | |
|---|---|---|---|---|---|---|---|---|---|---|---|
| | | | | | | | | | | | |
| | | | 0.7 | 0.7 | 0.7 | 0.7 | 0.7 | 0.7 | | | |
| | | 0.7 | 0.9 | 0.9 | 0.9 | 0.9 | 0.9 | 0.9 | 0.7 | | |
| | 0.7 | 0.9 | 0.9 | | | | | 0.9 | 0.9 | 0.7 | |
| | 0.7 | 0.9 | | | | | | | 0.9 | 0.7 | |
| | 0.7 | 0.9 | | | | | | | 0.9 | 0.7 | |
| | 0.7 | 0.9 | | | | | | | 0.9 | 0.7 | |
| | 0.7 | 0.9 | | | | | | | 0.9 | 0.7 | |
| | 0.7 | 0.9 | 0.9 | | | | | 0.9 | 0.9 | 0.7 | |
| | | 0.7 | 0.9 | 0.9 | 0.9 | 0.9 | 0.9 | 0.8 | 0.7 | | |
| | | | 0.7 | 0.7 | 0.7 | 0.7 | 0.7 | 0.7 | | | |
| | | | | | | | | | | | |

FIG. 14

FIG. 15

FIG. 16

| ELEMENT | MODEL TYPE |
|---|---|
| FILLER A | ELASTIC BODY<br>(VISCOSITY COEFFICIENT ZERO) |
| MATRIX PHASE B | PHASE FIELD MODEL |
| INTERFACE PHASE C | PHASE FIELD MODEL |

FIG. 17

| 1 | 1 | 1 | 1 | 1 | 1 | 1 | 1 | 1 | 1 | 1 | 1 |
|---|---|---|---|---|---|---|---|---|---|---|---|
| 1 | 1 | 1 | 0.8 | 0.8 | 0.8 | 0.8 | 0.8 | 0.8 | 1 | 1 | 1 |
| 1 | 1 | 0.8 | 0.8 | 0.8 | 0.8 | 0.8 | 0.8 | 0.8 | 0.8 | 1 | 1 |
| 1 | 0.8 | 0.8 | 0.8 | | | | | 0.8 | 0.8 | 0.8 | 1 |
| 1 | 0.8 | 0.8 | | | | | | | 0.8 | 0.8 | 1 |
| 1 | 0.8 | 0.8 | | | | | | | 0.8 | 0.8 | 1 |
| 1 | 0.8 | 0.8 | | | | | | | 0.8 | 0.8 | 1 |
| 1 | 0.8 | 0.8 | | | | | | | 0.8 | 0.8 | 1 |
| 1 | 0.8 | 0.8 | 0.8 | | | | | 0.8 | 0.8 | 0.8 | 1 |
| 1 | 1 | 0.8 | 0.8 | 0.8 | 0.8 | 0.8 | 0.8 | 0.8 | 0.8 | 1 | 1 |
| 1 | 1 | 1 | 0.8 | 0.8 | 0.8 | 0.8 | 0.8 | 0.8 | 1 | 1 | 1 |
| 1 | 1 | 1 | 1 | 1 | 1 | 1 | 1 | 1 | 1 | 1 | 1 |

## INTERNATIONAL SEARCH REPORT

| International application No. |
|---|
| **PCT/JP2022/042597** |

**A. CLASSIFICATION OF SUBJECT MATTER**

***G16C 20/30***(2019.01)i; ***G01N 3/00***(2006.01)i

FI: G16C20/30; G01N3/00 K

According to International Patent Classification (IPC) or to both national classification and IPC

**B. FIELDS SEARCHED**

Minimum documentation searched (classification system followed by classification symbols)

G16C20/30; G01N3/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-3747 A (YOKOHAMA RUBBER CO LTD) 08 January 2009 (2009-01-08) paragraphs [0004], [0016], [0020], [0021], [0023]-[0042], fig. 3, 5 | 1-15 |
| Y | 深堀 美英, ゴムのカーボン ブラック補強解明の新展開（下）, 日本ゴム協会誌, 2010, vol. 83, no. 6, pp. 182-189 in particular, "2.2", fig. 4, (FUKAHORI, Yoshihide. New Progress in the Carbon Black Reinforcement of Rubber (Part 2). NIPPON GOMU KYOKAISHI.) | 1-15 |
| Y | JP 2017-224098 A (BRIDGESTONE CORP) 21 December 2017 (2017-12-21) paragraphs [0028]-[0029] | 1-15 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 January 2023** | **17 January 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

| | International application No. |
|---|---|
| | **PCT/JP2022/042597** |

| Patent document cited in search report | | | Publication date (day/month/year) | Patent family member(s) | Publication date (day/month/year) |
|---|---|---|---|---|---|
| JP | 2009-3747 | A | 08 January 2009 | (Family: none) | |
| JP | 2017-224098 | A | 21 December 2017 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6637845 B